(19) **Europäisches Patentamt**
**European Patent Office**
**Office européen des brevets**

(11) **EP 2 806 817 B1**

(12) **EUROPEAN PATENT SPECIFICATION**

(45) Date of publication and mention
of the grant of the patent:
**16.09.2015 Bulletin 2015/38**

(51) Int Cl.:
*A61B 19/00* *(2006.01)* *B81B 1/00* *(2006.01)*
*A61B 17/00* *(2006.01)*

(21) Application number: **13710033.5**

(22) Date of filing: **24.01.2013**

(86) International application number:
**PCT/IB2013/050601**

(87) International publication number:
**WO 2013/111076 (01.08.2013 Gazette 2013/31)**

(54) **DEVICE AND METHOD FOR CONTROLLED ADHESION UPON MOIST SUBSTRATE**

VORRICHTUNG UND VERFAHREN ZUR KONTROLLIERTEN HAFTUNG AUF EINEM FEUCHTEN SUBSTRAT

DISPOSITIF ET PROCÉDÉ D'ADHÉSION CONTRÔLÉE SUR UN SUBSTRAT HUMIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.01.2012 IT RM20120026**

(43) Date of publication of application:
**03.12.2014 Bulletin 2014/49**

(73) Proprietor: **Universita' Campus Bio-Medico di Roma**
**00128 Rome (IT)**

(72) Inventors:
• **ACCOTO, Dino**
**73032 Andrano LE (IT)**

• **FRANCOMANO, Maria Teresa**
**00128 Roma RM (IT)**
• **ESPOSITO, Caterina**
**00195 Roma (IT)**

(74) Representative: **Papa, Elisabetta et al**
**Società Italiana Brevetti S.p.A**
**Piazza di Pietra, 39**
**00186 Roma (IT)**

(56) References cited:
**US-A- 2 983 273**     **US-A1- 2005 113 936**
**US-A1- 2005 119 640**     **US-A1- 2009 074 834**

## Description

**Technical Field of the Invention**

The present invention refers to a device for generating an adjustable adhesion force on a wet substrate, for instance in order to provide a control of the forces allowing locomotion on the latter of machines or other devices, or for a displacement or a manipulation of the substrate itself.

**Background**

Locomotion on a moist substrate and manipulation (e.g., lifting, translation, release) of items surrounded by moist surfaces require that, between surfaces in contact (e.g., between manipuland and manipulator), adequate adhesion forces be established and that the intensity of such forces be capable of being modulated.

For instance, in the bioengineering field such a requirement might be related to intracorporeal navigation of robots that move by exchanging forces with biological tissues with which they are in contact (e.g. gastrointestinal tract mucosae or the peritoneum surface). in the bioengineering field, such a requirement is related to the need to manipulate moist substrates of small-size objects, such as components of microelectro-mechanic systems. Said need may arise in industrial contexts for performing microassembling tasks, or tasks of pick-and-place type. In this latter application scenario, the moist surface is generally that of the manipulator. US2983273 discloses an electrode using capillary holding forces and is considered to be the closest prior art for the present invention.

The devices and methods used in the above-cited application fields entail limitations linked to the reduced possibility of controlling the adhesion force transmitted through the moist interface.

In the case, e.g., of the locomotion devices and techniques known in endoscopy, these did not satisfactorily solve the problem of control of device adhesion on the substrate, as instead necessary in order to be able to apply to the substrate itself a force suitable to produce the locomotor thrust.

Also as a consequence of this, in known devices for the generation of adhesion forces - and particularly in those for biomedical use - there are generally found:

- high mechanical complexity, which in turn prevents a radical miniaturization of the components, needed instead for numerous intracorporeal applications;
- low intrinsic safety, as the adhesion force can be generated through mechanic or pneumatic systems potentially capable of inflicting traumas or microtraumas to biological tissues;
- low level of controllability of the adhesion force;
- high manufacturing and assembly costs.

Always by way of example, in the field of manipulation systems, it is found that mechanic-type conventional manipulators run the risk of deforming and/or damaging manipulated components in case they are particularly delicate; such a problem is found, e.g., in the field of miniaturized componentry manipulation.

On the contrary, capillary forces can be exploited to obtain grip forces that are firm but not excessively oversized with respect to the weight of the manipulated component. Capillary micromanipulators exploit lifting forces of capillary bridges that form between two moist solid surfaces. One of the limitations of this type of lifters is that the adhesion force depends on the geometry of the liquid bridges and therefore generally decreases once the manipulated component has been lifted. Moreover, the control of the capillary force is generally of ON-OFF type, as the electro-induced necking down of the capillary bridges below a critical diameter entails their breakage and therefore manipuland detachment.

**Summary of the Invention**

The present invention provides a device and a method as defined in the appended claims allowing to obtain an adhesion capable of being modulated on a moist substrate, obviating some problems such as, e.g., those mentioned above in the field of locomotion and micromanipulation.

Control of adhesion on a moist substrate is obtained by a device according to claim 1 and, according to the same inventive concept, by a method according to claim 12.

Preferred features of the present invention are set forth in the dependent claims thereof.

In the present context, by "moist substrate" it is meant a generally solid substrate having a liquid, for instance an aqueous solution, on its surface. Hence, also a wet or liquid-imbibed substrate falls within the meaning of "moist".

The present invention provides some relevant advantages. The main advantage lies in that it enables to obtain an optimal adhesion, and therefore an optimal friction, on the wet substrate, both to the ends, e.g., of a locomotion and of the grip on the substrate itself. Moreover, such adhesion is capable of being modulated depending on contingent needs relative to the specific application.

Further advantages, features and the modes of employ of the present invention will be made evident in the following detailed description of some embodiments thereof, given by way of example and not for limitative purposes.

**Brief description of the Figures**

Reference will be made to the figures of the annexed drawings, wherein:

- Figure 1 shows a schematic illustration, in a cross section, of the mechanism of the so-called "wet adhesion" involving two fluids, in particular two liquids;

- Figure 2 schematically illustrates, in a cross section, the phenomenon of the so-called "electrowetting", in which the angle of wettability of a liquid on a solid substrate is varied by the applying of a voltage;

- Figures 3A to 3B show each a schematic view, in a cross section, of a device according to a preferred embodiment of the invention, during the applying of an electric field that progressively decreases the adhesion force at the interface of the device with a moist surface; and

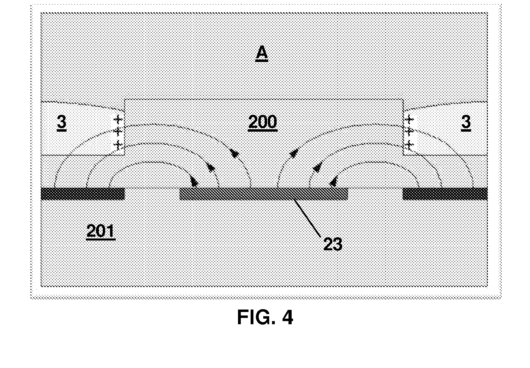

- Figure 4 shows an enlarged detail of Figure 3B, in which the electric field lines and the corresponding polarization of an adhesion surface of the device are exemplarily shown.

**Detailed description of preferred embodiments**

Preliminarily to the detailed description of preferred embodiments of the device of the invention, the theoretical bases of the invention itself are illustrated hereinafter.

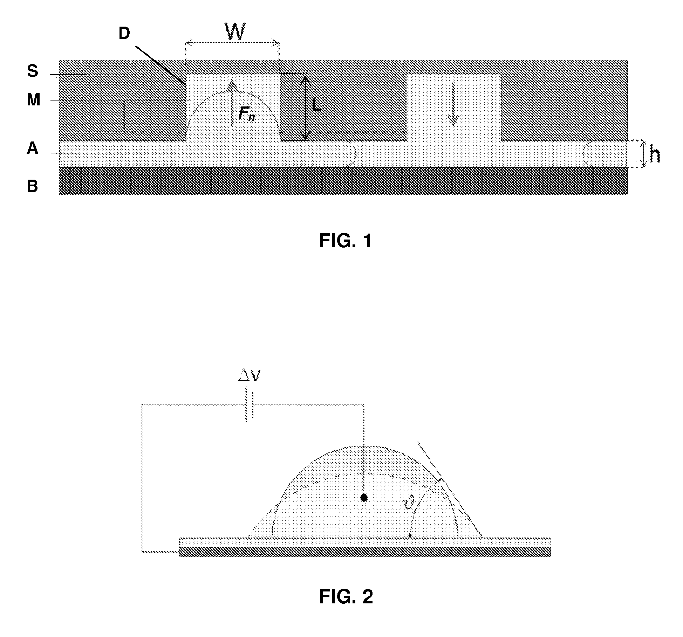

Figure 1 schematically illustrates the principle of the so-called "wet adhesion", present in nature and used, e.g., by some Amphibians belonging to the Ranidae family for stationing on a wet substrate.

In said figure a movable surface S is shown - corresponding, in said example, to the bottom part of the amphibian legs - bearing surface discontinuities D, shown purely by way of example in the form of channels of defined and regular geometry, but that in nature are of irregular geometry.

The movable surface S generates a mucus M and rests on a moist external surface or substrate B, the latter in particular wet by a liquid A, typically water.

Wet adhesion of the movable surface S on the external surface B is regulated, in the example considered, by controlling the discharge of mucus M and therefore, ultimately, the capillary return of water A within the discontinuities D.

Said phenomenon is described by equations:

$$\Delta p = 2\gamma\left(\frac{1}{h} - \frac{1}{W}\right)$$

and

$$F_n = \Delta p b W = 2\gamma b W\left(\frac{1}{h} - \frac{1}{W}\right)$$

wherein:

- $\Delta p$ defines the pressure difference at the mucus M - water A interface between the two fluids;
- $F_n$ represents the normal force at the mucus M - water A interface, returning the water meniscus A within the discontinuity D;
- $\gamma$ represents the coefficient of surface tension at said interface between fluids A and M;
- $W$ represents the width of the channel D;
- $h$ represents the distance between the movable surface S and the external surface B; and
- $b$ defines the longitudinal dimension (depth) of the discontinuity, in a direction orthogonal to the cross section illustrated in Figure 1.

Figure 2 schematizes the phenomenon known as "electrowetting", showing how the wettability of a fluid with respect to a surface - the latter solid or it also fluid - may be varied by the applying of an electric voltage.

In particular, the angle of wettability, $\vartheta$, illustrated in Figure 2, varies with the applying of an electric potential. In the example shown, said angle $\vartheta$ increases with the applying of the potential ($\Delta V$), and therefore decreases the wettability proper of the fluid on the surface. Of course, it is also possible to produce an opposite effect, i.e. decrease the angle of contact (increase the wettability) by applying an appropriate potential.

The electrowetting phenomenon is linked to the variation of the coefficient of surface tension ($\gamma$) of the liquid-air interface following the applying of a potential (V). Such variation is described by the so-called Lippmann equation:

$$\left(\frac{\partial\gamma}{\partial V}\right)_{p,T,\mu} = -\sigma$$

wherein:

- $p$, $T$ and $\mu$ respectively represent pressure, temperature and chemical potential; and
- $\sigma$ is the charge density on the surface of the liquid.

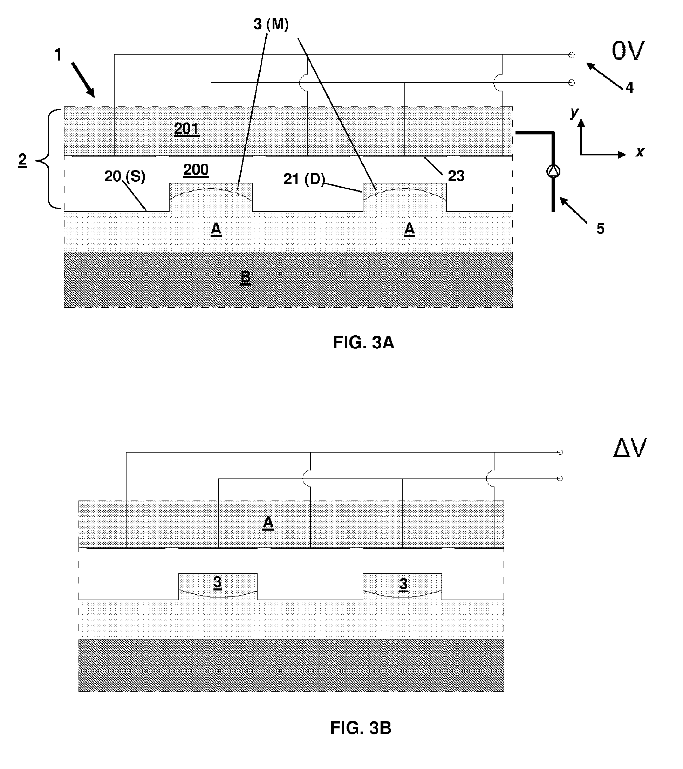

In the embodiment of the invention described hereinafter, referring to Figures 3A and 3B, the wettability of an interface liquid on a surface - corresponding respectively to the mucus M and the movable surface S in the above-considered example, with reference to Figure 1 - is modified, and in particular decreased, by the applying of an electric field. The varied wettability of said interface fluid entails a modification in the capillary return of a substrate liquid within the surface - liquid and substrate corresponding respectively to water A and to the external surface B in the example of Figure 1.

Referring initially to Figure 3A, a device apt to generate an adjustable adhesion force on a substrate or moist surface B according to a preferred embodiment of the invention is generally denoted by 1.

The substrate B is, as mentioned above, moist, bearing in particular a liquid, which will be referred to as substrate liquid A. In the present example, the device 1 may be construed as part of a robotic capsule equipped with a locomotion system for navigation in the intestine. In these particular cases, the substrate B can represent the intestinal wall, or mucosa, and the substrate liquid A the mucosa-secreted mucus, respectively.

The device 1 comprises a main body generally denoted by 2, bearing a surface 20, which will be referred to as adhesion surface and that corresponds to the movable surface S of the example of Figure 1, illustrated above.

The adhesion surface 20, in use, arranges itself facing the substrate B.

The adhesion surface 20 bears a plurality of recesses 21, corresponding to the discontinuities D of Figure 1, each apt to generate a capillary return for the substrate fluid **A.** In the present example such recesses 21 are in the form of channels extending the one parallel to the other one along the longitudinal development of the adhesion surface 20, i.e. orthogonally to the plane of the cross-section shown in Figure 3A.

As already in the example of Figure 1, each channel has a cross section of substantially rectangular shape. The planar dimensions of said cross section are denotable with L and W and develop respectively along an axis y, defining a normal direction of approach of the adhesion surface 21 to the substrate surface B, and along a direction x orthogonal thereto.

Preferably, the main body 2 has a first adhesion portion 200, bearing precisely the adhesion surface 21, and a second support portion 201, fixed to the first portion 200 and transversally side-by-side thereto along the above-defined direction *y*.

Preferably, the first portion 200 of the main body 2 is made of polymeric material, e.g. polydimethylsiloxane (PDMS).

Electrodes 23 for the applying of an electric field are arranged at the interface between the two portions 200 and 201 of the main body 2. In the present example, each electrode 23 is of flat configuration, with a rectangular plan. The electrode 23 extends longitudinally in the main body 2, at and parallelly to a respective channel 21 or at and parallelly to the projection interposed between contiguous channels. Always in the present example, the electrodes 23 are equidistant the one from the other along direction x and have a regular arrangement. Preferably, the electrodes 23 are of noble metal, e.g. platinum.

The electrodes 23 are part of electric field generating means, generally denoted by 4. In the present example, such means 4 is suitable to generate a static electric field, with potential difference adjustable according to specific adhesion needs, as will be illustrated in greater detail shortly.

The device 1 further comprises fluid delivery and/or reservoir means, generally denoted by 5 and only schematically shown in Fig. 3A. The delivery and/or reservoir means 5 is apt to provide an interface fluid 3 at the adhesion surface 20, so that said fluid, in use, arranges itself interposed between the adhesion surface itself and the substrate fluid A. The interface fluid 3 therefore corresponds to the mucus M of the example of Figure 1. Preferably, the delivery and/or reservoir means is obtained by capillary confining of the related fluid, e.g. at the recesses 21 of the surface 20.

In more general terms, fluid delivery and/or reservoir means may be provided, apt to ensure the presence of the aforesaid interface fluid 3 at the adhesion surface 20. In a particularly advantageous embodiment of the invention, the interface fluid 3 is a silicone oil.

Exemplary dimensions of the various component of the device 1 are indicated in the following, with reference to Figures 3A and 3B.

- Substrate 201 - surface area (76 x 24) mm$^2$; thickness (y direction) 1.1 mm.

- Electrodes 23 - length (longitudinal direction orthogonal to that of the section of Figure 3A and 3B) = 15.5 mm, thickness (y direction) = 20 nm, width (x direction) = 150 $\mu$m.

- Adhesion layer 200 - covers an area equal to (45 x 15) mm$^2$; in particular, each channel has a length (longitudinal direction orthogonal to that of the section of Figure 3A and 3B) = 15 mm, a width (*W*) = 150 $\mu$m and a height (L) = 50 $\mu$m; the residual thickness of the layer at each channel is of 50 $\mu$m (at the channel walls, the polymer has an overall thickness = 100 $\mu$m).

- substrate liquid M - the substrate 200 is coated with 0.1 ml of substrate liquid M, preferably silicone oil.

Silicone oils have the following preferred properties: i) availability of wide ranges of kinematic viscosity; and ii) good wettability in comparison with the substrate 200, e.g. attained by way of their chemical affinity.

With the above-indicated dimensional and tribological data, a preferred static electric field envisages the applying of a potential difference of about 60 V at the ends of adjacent electrodes 23 , as shown in Figure 3A.

The modes of employ of the device 1 described hereto will presently be illustrated.

In Figure 3A, the device 1 is shown in the configuration in which there is no electric field applied. The adhesion surface 20 is at an exemplary distance *h* from the substrate B. The distance *h* will have a maximum value of the order of the tens of micron.

Under such circumstances, the silicone oil 3 has high wettability with respect to the adhesion surface 20, as is inferred by the angles of wettability shown. In particular, the meniscus of the silicone oil 3 has a substantially concave profile.

Under said condition, the substrate fluid A is returned by capillarity within the channels 21. Therefore, the "wet adhesion" phenomenon is established, in which a normal adhesion force ($F_n$) is established at the interface between the two fluid phases, silicone oil 3 - substrate liquid A.

In Figure 3B, the device 1 is shown with the applying of an electric field corresponding to a potential difference ΔV. Under said condition, the angle of wettability of the silicone oil 3 with respect to the adhesion surface 20 increases, i.e. the wettability of the surface 20 reduces. The variation of curvature of the meniscus produces an effect on the intensity of the normal force $F_n$. In the illustrated case, $F_n$ tends to decrease.

Referring to the example of use of the device 1 in intracorporeal locomotion, the adhesion condition of Figure 3A enables to exert a firm grip of the device 1 on the biological tissue, e.g. on the intestinal mucosa, to the ends of a controlled stop or slowing down.

In the next step, shown in Figure 3B, the device can detach from the tissue to advance in the body, e.g. driven by peristaltic waves.

A possible advantageous application of the device 1, in the field of intracorporeal locomotion, would see it employed on the rotatable members of the locomotion device according to the "pinch locomotion" principle of which at the Italian Pat. Appln. No. RM2009A000635 published as ITRM20090635.

On the basis of a hereto-mentioned variant embodiment, the device of the invention may envisage that the electric field be applied to produce an increase rather than a decrease of wettability of the interface fluid on the adhesion surface. Said variant may envisage the use of a suitable combination of materials for the manufacturing of the moist and adhesion substrates, as well as for the fluid 3.

Moreover, referring to the use of the device 1 for robot locomotion, the possible application thereof in industrial contexts different from the above-mentioned biomedical ones, e.g. for navigation in artificial or natural ducts or lumens, is highlighted.

The device is also particularly suitable for underwater use or for development of end effectors of manipulators.

As mentioned above, the invention further provides a method for generating an adjustable adhesion force between an adhesion surface as defined above and a wet substrate, mutually facing. The method proposed mainly comprises:

- providing a plurality of recesses, as those defined above, on the adhesion surface, which are apt to generate a capillary return for a substrate fluid as introduced above;

- providing an interface fluid at the adhesion surface, so that this interface fluid, in use, arranges itself interposed between the adhesion surface and the substrate fluid; and

- selectively generating an electric field at the adhesion surface, so as to modify the wettability of the interface fluid on the adhesion surface.

The preferred features of the method have already been illustrated above with reference to the device 1 and its variants.

The present invention has been hereto described with reference to preferred embodiments thereof. It is understood that other embodiments might exist, all falling within the concept of the same invention, as defined by the protective scope of the claims hereinafter.

**Claims**

1. A device (1) for generating an adjustable adhesion force on a wet substrate (B), in particular in order to provide a locomotion of the device or a manipulation of the substrate (B), comprising:

   - a main body (2) having an adhesion surface (20) which, in use, is arranged facing the substrate, wherein at such adhesion surface (20) said main body (2) has a plurality of recesses (21) apt to generate a capillary return for a substrate fluid (A) present on the substrate;
   **characterized in** further comprising
   - fluid delivery and/or reservoir means (5), apt to provide an interface fluid (3) at said adhesion surface (20), so that the interface fluid (3), in use, is arranged interposed between the adhesion surface (20) and the substrate fluid; and
   - electric field generating means (4), apt to generate an electric field at the adhesion surface (20), so as to modify the wettability of the interface fluid (3) on said adhesion surface (20).

2. The device (1) according to claim 1, wherein the adhesion surface (20) has a plurality of channels (21) extending longitudinally thereon.

3. The device (1) according to the preceding claim, wherein the channels (21) have a substantially squared cross-section, preferably rectangular.

4. The device (1) according to claim 2 or 3, wherein the normal section of each channel has the following dimensions: depth L = about 50 μm and width W = about 150 μm.

5. The device (1) according to any one of the preceding claims, wherein the electric field generating means (4) comprises a plurality of electrodes (23), preferably of noble metal, housed within the main body (2), wherein the electrodes (23) preferably have a flat configuration, preferably with a rectangular plan.

6. The device (1) according to the preceding claim, wherein the electrodes (23) have a regular arrangement.

7. The device (1) according to any one of the preceding claims, wherein the electric field generating means (4) is apt to generate a static electric field.

8. The device (1) according to any one of the preceding claims, wherein the main body (2) is made, at least at said adhesion surface (20), of polydimethylsiloxane (PDMS).

9. The device (1) according to any one of the preceding claims, wherein the main body is made, at least at a portion (201) supporting said adhesion surface (20), of glass or other dielectric material, such as polymers.

10. The device (1) according to any one of the preceding claims, wherein the interface fluid is a silicone oil (3).

11. The device (1) according to any one of the preceding claims, wherein the overall arrangement is such that the applying of the electric field produces a variation of wettability of said interface fluid (3) on the adhesion surface (20).

12. A method for generating an adjustable adhesion force between an adhesion surface (20) and a wet substrate (B) mutually facing, for instance to the ends of a locomotion of a navigation and/or manipulation device (1) bearing such adhesion surface (20), which method comprises:

   - providing a plurality of recesses (21) on said adhesion surface (20), which recesses (21) are apt to generate a capillary return for a substrate fluid (A) present on the latter;
   **characterized in** further comprising:
   - providing an interface fluid (3) at said adhesion surface (20), so that said interface fluid (3), in use, arranges itself interposed between the adhesion surface (20) and the substrate fluid; and
   - selectively generating an electric field at the adhesion surface (20), so as to modify the wettability of the interface fluid (3) on the adhesion surface (20).

13. The method according to claim 12, wherein on the adhesion surface (20) a plurality of channels (21) is provided, arranged along a geometric regular pattern.

14. The method according to claim 12 or 13, wherein the generation of a static electric field is provided.

15. The method according to any one of the claims 12 to 14, wherein the interface fluid is a silicone oil (3).

16. The method according to any one of the claims 12 to 15, wherein the overall arrangement is such that the applying of the electric field produces a decrease or an increase of wettability of said interface fluid (3) on said adhesion surface (20).

**Patentansprüche**

1. Vorrichtung (1) zur Erzeugung einer justierbaren Haftkraft auf einem feuchten Substrat (B), im Besonderen um eine Fortbewegung der Vorrichtung oder eine Manipulation des Substrats (B) zur Verfügung zu stellen, die umfasst:

   - einen Hauptkorpus (2), der über eine Haftoberfläche (20) verfügt, die im Einsatz geeignet ist gegenüber dem Substrat angeordnet zu sein, wobei bei einer solchen Haftoberfläche (20) der Hauptkorpus (2) über eine Mehrzahl von Aussparungen (21) verfügt, die geeignet sind einen Kapillarrücklauf für eine Substratflüssigkeit (A) zu erzeugen, die auf dem Substrat vorhanden ist;
   **dadurch gekennzeichnet, dass** sie weiterhin umfasst:
   - Flüssigkeitszufuhr- und/oder -reservoirmittel (5), die geeignet sind eine Schnittstellenflüssigkeit (3) bei der Haftoberfläche (20) zur Verfügung zu stellen, so dass die Schnittstellenflüssigkeit (3) im Einsatz zwischen der Haftoberfläche (20) und der Substratflüssigkeit angeordnet ist; und
   - Mittel (4) zur Erzeugung eines elektrischen Feldes, das geeignet ist ein elektrisches Feld bei der Haftoberfläche (20) zu erzeugen, um so die Benetzbarkeit der Schnittstellenflüssigkeit (3) auf der Haftoberfläche (20) zu modifizieren.

2. Vorrichtung (1) gemäß Anspruch 1, wobei die Haftoberfläche (20) über eine Mehrzahl von Kanälen (21) verfügt, die sich darauf longitudinal erstrecken.

3. Vorrichtung (1) gemäß dem vorangehenden Anspruch, wobei die Kanäle (21) über einen im Wesentlichen quadratischen, vorzugsweise rechteckigen, Querschnitt verfügen.

4. Vorrichtung (1) gemäß Anspruch 2 oder 3, wobei der Normalschnitt eines jeden Kanals über die folgenden Dimensionen verfügt: Tiefe L = ungefähr 50 $\mu$m und Breite W = ungefähr 150 $\mu$m.

5. Vorrichtung (1) gemäß einem der vorangehenden Ansprüche, wobei das Mittel (4) zur Erzeugung eines elektrischen Feldes umfasst: eine Mehrzahl von Elektroden (23), vorzugsweise aus Edelmetall, die innerhalb des Hauptkorpus (2) untergebracht sind, wobei die Elektroden (23) vorzugsweise über eine flache Konfiguration verfügen, vorzugsweise mit einer rechteckigen Fläche.

6. Vorrichtung (1) gemäß dem vorangehenden Anspruch, wobei die Elektroden (23) über eine regelmäßige Anordnung verfügen.

7. Vorrichtung (1) gemäß einem der vorangehenden Ansprüche, wobei das Mittel (4) zur Erzeugung eines elektrischen Feldes geeignet ist ein statisches elektrisches Feld zu erzeugen.

8. Vorrichtung (1) gemäß einem der vorangehenden Ansprüche, wobei der Hauptkorpus (2), mindestens bei der Haftoberfläche (20), aus Polydimethylsiloxan (PDMS) besteht.

9. Vorrichtung (1) gemäß einem der vorangehenden Ansprüche, wobei der Hauptkorpus mindestens bei einem Teil (201), der die Haftoberfläche (20) unterstützt, aus Glas oder einem anderen dielektrischen Material, wie zum Beispiel Polymeren, besteht.

10. Vorrichtung (1) gemäß einem der vorangehenden Ansprüche, wobei die Schnittstellenflüssigkeit ein Silikonöl (3) ist.

11. Vorrichtung (1) gemäß einem der vorangehenden Ansprüche, wobei die Gesamtanordnung so ist, dass das Anlegen des elektrischen Feldes eine Veränderung der Benetzbarkeit der Schnittstellenflüssigkeit (3) auf der Haftoberfläche (20) erzeugt.

12. Verfahren zur Erzeugung einer justierbaren Haftkraft zwischen einer Haftoberfläche (20) und einem feuchten Substrat (B), die wechselseitig gegenüberliegend angeordnet sind, zum Beispiel zum Ende einer Fortbewegung einer Navigations- und/oder Manipulationsvorrichtung (1), die eine solche Haftoberfläche (20) tragen, wobei das Verfahren umfasst:

- Bereitstellung einer Mehrzahl von Aussparungen (21) auf der Haftoberfläche (20), wobei die Aussparungen (21) geeignet sind einen Kapillarrücklauf für eine Substratflüssigkeit (A) zu erzeugen, die auf letzterer vorhanden ist; **dadurch gekennzeichnet, dass** das Verfahren weiterhin umfasst:
- Bereitstellung einer Schnittstellenflüssigkeit (3) bei der Haftoberfläche (20), so dass sich die Schnittstellenflüssigkeit (3) im Einsatz selbst zwischen die Haftoberfläche (20) und die Schnittstellenflüssigkeit anordnet; und
- selektive Erzeugung eines elektrischen Feldes bei der Haftoberfläche (20), um so die Benetzbarkeit der Schnittstellenflüssigkeit (3) auf der Haftoberfläche (20) zu modifizieren.

13. Verfahren gemäß Anspruch 12, wobei auf der Haftoberfläche (20) eine Mehrzahl von Kanälen (21) zur Verfügung gestellt wird, die entlang eines regelmäßigen geographischen Musters angeordnet sind.

14. Verfahren gemäß Anspruch 12 oder 13, wobei die Erzeugung eines statischen elektrischen Feldes zur Verfügung gestellt wird.

15. Verfahren gemäß einem der Ansprüche 12 bis 14, wobei die Schnittstellenflüssigkeit ein Silikonöl (3) ist.

16. Verfahren gemäß einem der Ansprüche 12 bis 15, wobei die Gesamtanordnung so ist, dass das Anlegen des elektrischen Feldes eine Abnahme oder eine Zunahme der Benetzbarkeit der Schnittstellenflüssigkeit (3) auf der Haftoberfläche (20) erzeugt.

## Revendications

1. Dispositif (1) pour générer une force d'adhésion ajustable sur un substrat humide (B), en particulier pour fournir la locomotion du dispositif ou une manipulation du substrat (B), comprenant :

- un corps principal (2) ayant une surface d'adhésion (20) qui, à l'usage, est agencée face au substrat, dans lequel au niveau d'une telle surface d'adhésion (20), ledit corps principal (2) a une pluralité d'évidements (21) aptes à générer un retour capillaire pour un fluide de substrat (A) présent sur le substrat ;

**caractérisé en ce qu'**il comprend en outre :

- des moyens de distribution de fluide et/ou de réservoir (5) aptes à fournir un fluide d'interface (3) au niveau de ladite surface d'adhésion (20), de sorte que le fluide d'interface (3), à l'usage, est agencé en étant intercalé entre la surface d'adhésion (20) et le fluide de substrat ; et
- des moyens de génération de champ électrique (4) aptes à générer un champ électrique au niveau de la surface d'adhésion (20), afin de modifier la mouillabilité du fluide d'interface (3) sur ladite surface d'adhésion (20).

2. Dispositif (1) selon la revendication 1, dans lequel la surface d'adhésion (20) a une pluralité de canaux (21) s'étendant longitudinalement sur cette dernière.

3. Dispositif (1) selon la revendication précédente, dans lequel les canaux (21) ont une section transversale sensiblement carrée, de préférence rectangulaire.

4. Dispositif (1) selon la revendication 2 ou 3, dans lequel la section normale de chaque canal a les dimensions suivantes : profondeur L = environ 50 $\mu$m et largeur W = environ 150 $\mu$m.

5. Dispositif (1) selon l'une quelconque des revendications précédentes, dans lequel les moyens de génération de champ électrique (4) comprennent une pluralité d'électrodes (23), de préférence un métal noble, logées à l'intérieur du corps principal (2), dans lequel les électrodes (23) ont de préférence une configuration plate, de préférence avec un plan rectangulaire.

6. Dispositif (1) selon la revendication précédente, dans lequel les électrodes (23) ont un agencement régulier.

7. Dispositif (1) selon l'une quelconque des revendications précédentes, dans lequel les moyens de génération de champ électrique (4) sont aptes à générer un champ électrique statique.

8. Dispositif (1) selon l'une quelconque des revendications précédentes, dans lequel le corps principal (2) est réalisé, au moins au niveau de ladite surface d'adhésion (20), avec du polydiméthylsiloxane (PDMS).

9. Dispositif (1) selon l'une quelconque des revendications précédentes, dans lequel le corps principal est réalisé, au moins au niveau d'une partie (201) supportant ladite surface d'adhésion (20), avec du verre ou un autre matériau diélectrique, tels que les polymères.

10. Dispositif (1) selon l'une quelconque des revendications précédentes, dans lequel le fluide d'interface est une huile de silicone (3).

11. Dispositif (1) selon l'une quelconque des revendications précédentes, dans lequel l'agencement global est tel que l'application du champ électrique produit une variation de mouillabilité dudit fluide d'interface (3) sur la surface d'adhésion (20).

12. Procédé pour générer une force d'adhésion ajustable entre une surface d'adhésion (20) et un substrat humide (B) se faisant mutuellement face, par exemple, aux fins de la locomotion d'un dispositif de navigation et/ou de manipulation (1) supportant une telle surface d'adhésion (20), ledit procédé comprend l'étape consistant à :

- prévoir une pluralité d'évidements (21) sur ladite surface d'adhésion (20), lesquels évidements (21) sont aptes à générer un retour capillaire pour un fluide de substrat (A) présents sur ces derniers ;

**caractérisé en ce qu'**il comprend en outre les étapes consistant à :

- prévoir un fluide d'interface (3) au niveau de ladite surface d'adhésion (20), de sorte que ledit fluide d'interface (3), à l'usage, est agencé de lui-même en étant intercalé entre la surface d'adhésion (20) et le fluide de substrat ; et
- générer sélectivement un champ électrique au niveau de la surface d'adhésion (20), afin de modifier la mouillabilité du fluide d'interface (3) sur la surface d'adhésion (20).

13. Procédé selon la revendication 12, dans lequel sur la surface d'adhésion (20), on prévoit une pluralité de canaux (21) agencés le long d'un modèle géométrique régulier.

14. Procédé selon la revendication 12 ou 13, dans lequel on prévoit la génération d'un champ électrique statique.

15. Procédé selon l'une quelconque des revendications 12 à 14, dans lequel le fluide d'interface est une huile de silicone (3).

16. Procédé selon l'une quelconque des revendications 12 à 15, dans lequel l'agencement global est tel que l'application du champ électrique produit une diminution ou une augmentation de mouillabilité dudit fluide d'interface (3) sur ladite surface d'adhésion (20).

**FIG. 1**

**FIG. 2**

FIG. 3A

FIG. 3B

FIG. 4

**EP 2 806 817 B1**

**Patent documents cited in the description**

- US 2983273 A
- IT RM20090635 A
- IT RM20090635